(19) Europäisches Patentamt / European Patent Office / Office européen des brevets

(11) **EP 1 923 015 B1**

(12) **EUROPÄISCHE PATENTSCHRIFT**

(45) Veröffentlichungstag und Bekanntmachung des Hinweises auf die Patenterteilung:
**08.07.2009 Patentblatt 2009/28**

(51) Int Cl.:
*A61B 19/00* (2006.01)    *G06F 19/00* (2006.01)

(21) Anmeldenummer: **06021696.7**

(22) Anmeldetag: **17.10.2006**

(54) **Markernavigationssystem zum Erfassen und Darstellen der Lage von Markereinrichtungen**

Navigation system with markers for obtaining and depicting said markers' position

Système de navigation avec marquers pour obtenir et representer leur position

(84) Benannte Vertragsstaaten:
**DE FR GB**

(43) Veröffentlichungstag der Anmeldung:
**21.05.2008 Patentblatt 2008/21**

(73) Patentinhaber: **BrainLAB AG**
**85622 Feldkirchen (DE)**

(72) Erfinder:
• **Maier, Christian**
**80802 München (DE)**
• **Uhde, Jörg**
**80538 München (DE)**
• **Weiser, Manfred**
**81673 München (DE)**

(74) Vertreter: **Schwabe - Sandmair - Marx**
**Patentanwälte**
**Stuntzstrasse 16**
**81677 München (DE)**

(56) Entgegenhaltungen:
| | |
|---|---|
| **EP-A1- 1 380 266** | **US-A- 5 749 362** |
| **US-A- 5 921 992** | **US-A1- 2003 088 179** |
| **US-A1- 2003 179 856** | **US-B1- 6 466 815** |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**EP 1 923 015 B1**

**Beschreibung**

Die vorliegende Erfindung betrifft ein Markernavigationssystem zum Erfassen und Darstellen der Lage von Markereinrichtungen. Die Markereinrichtungen sind insbesondere an Körperstrukturen, wie zum Beispiel Knochen oder künstlichen Gliedmaßen und/oder an Instrumenten, insbesondere medizinischen Instrumenten angebracht.

Herkömmliche Markernavigationssysteme, insbesondere so genannte Image-Guided-Surgery-Systeme (IGS-Systeme), detektieren und verfolgen üblicherweise die Lagen von einem oder mehreren Bezugssystemen, die beispielsweise mit einer Körperstruktur (z.B. Knochen) oder mit einem Instrument verbunden sind.

Üblicherweise zeigt die Anzeige eines Markernavigationssystems nur einen bestimmten Ausschnitt aus dem Operationsbereich. Ergibt sich eine Änderung im gezielten Ausschnitt, ist dabei problematisch, dass unklar ist, ob sich die Kamera bewegt hat oder ob eine Bewegung der beobachteten Markereinrichtungen stattgefunden hat, die beispielsweise mit einer Körperstruktur oder einem Instrument verbunden sind. Problematisch ist weiterhin, dass größere Relativbewegungen der Markereinrichtungen auftreten können, so dass die mit der jeweiligen Markereinrichtung verbundenen Gegenstände (z.B. Körperstruktur oder Instrument), die üblicherweise kalibriert sind, nicht mehr auf der Anzeige angezeigt werden, sondern außerhalb des gezeigten Ausschnitts (Anzeigebereichs) geraten sind. Letzteres liegt insbesondere daran, dass typischerweise nur ein den Operateur interessierender Ausschnitt aus dem gesamten, von der Kamera erfassten Raum gezeigt wird. Verlassen also die angezeigten, den Operateur interessierenden Gegenstände den gezeigten Ausschnitt (Anzeigebereich), so wünscht der Operateur, dass der Anzeigebereich (gezeigter Ausschnitt) so nachjustiert wird, dass die interessierenden Gegenstände wieder angezeigt werden.

Aufgabe der Erfindung ist es die Anzeige der Bewegung von Markereinrichtungen und/oder damit verbundenen Gegenständen, die mittels der Markereinrichtungen detektiert werden, so zu optimieren, dass eine Nachjustage des Anzeigebereichs weniger häufig erforderlich ist und/oder absolute Bewegungen mehrerer mit Markereinrichtungen versehener Gegenstände (Fragmente) in einem Bezugssystem (z.B. Kamerasystem) erfasst und dargestellt werden können.

Vorstehende Aufgabe wird erfindungsgemäß durch die Gegenstände der unabhängigen Ansprüche gelöst. Vorteilhafte Weiterbildungen gehen aus den abhängigen Ansprüchen hervor.

Vorteilhaft ermöglicht eine erfindungsgemäße Kompensation einer Drift angezeigter Gegenstände, eine informativere Darstellung der Markereinrichtungen und der mit ihnen verbundenen Gegenstände (z.B. Knochenfragmente) in einem Bezugssystem (z.B. Kamera-Bezugssystem). Insbesondere erreichen die Erfinder damit erstmalig ein unabhängiges Verfolgen (Tracking) von mehreren Fragmenten. Bewegt sich beispielsweise gemäß einem Fall 1 ein Fragment A nach oben, so wird angezeigt dass sich das Fragment A hebt. Bewegt sich gemäß einem Fall 2 das Fragment B nach oben, so wird angezeigt, dass sich das Fragment B hebt. Gemäß dem Stand der Technik war dies nicht möglich. An einem der Fragmente A und B wurde das Bezugssystem gebunden, es war also beispielsweise das Fragment A das Bezugssystem für die Darstellung und blieb in dieser gewählten Darstellung stets unbewegt. Die eben beschriebenen Fälle (Fall 1 und Fall 2) unterschieden sich dann dadurch, dass im Fall 1 sich das Fragment B senkt und sich im Fall 2 das Fragment B hebt. Berücksichtigt man beispielsweise einen virtuellen Massenschwerpunkt der Fragmente A und B, so ermöglicht auch dies nicht eine Darstellung der Bewegung der Fragmente in einem Bezugssystem, das beispielsweise im Operationssaal oder in dem (im Operationssaal ruhenden) Kamera-Bezugssystem ruht. Vorteilhaft erlaubt somit die vorliegende Erfindung die absolute Bewegung mehrerer Fragmente in einem Bezugssystem zu erfassen und darzustellen, das insbesondere im Kamera-Bezugssystem ruht und/oder im Raum ruht, in dem sich der Benutzer aufhält. Die erfindungsgemäße Driftkompensation hilft bei der Erzielung dieses Vorteils, stellt aber auch für sich alleine genommen einen wesentlichen Vorteil der Erfindung dar. Erfindungsgemäß werden mittels einer Detektionseinrichtung (z. B. Kamera oder Ultraschalldetektor) Markereinrichtungen detektiert. Die Markereinrichtungen umfassen typischerweise zwei bis vier Marker, die in fester und vorbestimmter relativer Lage zueinander angeordnet sind und insbesondere mechanisch verbunden sind. Die Marker können passive oder aktive Marker sein, wobei die passiven Marker Signale (z.B. Wellen und/oder Strahlung) reflektieren, die in ihre Richtung ausgesendet werden und die aktiven Marker selbst Ursprung der Signale (z.B. Strahlung und/oder Wellen) sind. Die von den (aktiven oder passiven) Markern ausgehenden Signale, bei denen es sich z.B. um Wellensignale oder Strahlungssignale handeln kann, werden von einer Detektionsvorrichtung (z. B. Kamera) detektiert. Um eine Position der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen.

Die Lage der Markereinrichtung wird bevorzugt durch die Position der Markereinrichtung in einem vorbestimmen Bezugssystem bestimmt. Vorzugsweise wird als Bezugssystem ein Bezugssystem verwendet, in dem die Detektionseinrichtung ruht. Insbesondere wird die Lage der Markereinrichtung durch die Positionen der Marker, insbesondere der Mittelpunkte der Marker in einem Bezugssystem bestimmt. Die Positionen können zum Beispiel mit kartesischen Koordinaten oder Kugelkoordinaten beschrieben werden. Die relative Lage von einem Teil (z. B. Detektionseinrichtung oder Markereinrichtung) zu einem anderen Teil (z. B. Markereinrichtung) kann insbesondere durch Raumwinkel und/oder Abstände und/oder Koordinaten (in einem Bezugssystem) und/oder Vektoren beschrieben werden und wird vorzugsweise aus dem die Lage beschreibenden Positionen z. B. mittels eines Programms errechnet, das auf einem Computer läuft.

Der hierin verwendete Begriff "relative Lage" oder der Ausdruck "Lage eines Teils A relativ zu einem Teil B" umfasst also den Begriff der relativen Positionen zwischen den zwei Teilen, insbesondere zwischen den Markereinrichtungen und/oder deren Markern oder zwischen einer Markereinrichtung (oder deren Markern) und der Detektionseinrichtung. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Die Markereinrichtung gemäß der vorliegenden Erfindung umfasst also vorzugsweise mindestens zwei Marker, insbesondere und bevorzugt drei Marker und können natürlich auch mehr als drei Marker umfassen. Die Abmessungen der Marker und die relativen Lagen der Marker zueinander sind bekannt und liegen insbesondere als vorbekannte Daten einer Datenverarbeitungseinrichtung vor oder wurden durch einen Kalibriervorgang zur Laufzeit des Datenverarbeitungsprogramms bestimmt. Vorzugsweise ist auch die Form der Marker bekannt.

Weiter umfasst das erfindungsgemäße Markernavigationssystem vorzugsweise eine Detektionseinrichtung, die Signale von den mindestens zwei Markern, insbesondere von mindestens 2 Markereinrichtungen (mit jeweils mindestens 2 Markern) detektiert. Wie zuvor ausgeführt handelt es sich hierbei um von den Markern ausgehende Signale, die entweder aktiv von den Markern ausgesendet werden oder von den Markern reflektiert werden. Im letzteren Fall ist vorzugsweise weiter eine Signalsendequelle beispielsweise eine Infrarotlichtquelle vorgesehen, die Signale (z. B. Ultraschallwellen oder Infrarotlicht) in Richtung auf die passiven Marker (kontinuierlich oder gepulst) aussendet, wobei die passiven Marker die Signale reflektieren. Eine Datenverarbeitungseinrichtung, insbesondere ein Computer erlaubt die Berechnung der relativen Lage der Markereinrichtung relativ zur Detektionseinrichtung, insbesondere die Berechnung der Lage der Markereinrichtung in einem Bezugssystem, in dem die Detektionseinrichtung ruht, also beispielsweise in einem Bezugssystem, das in einem Operationssaal ruht.

Die erfindungsgemäße Datenverarbeitungseinrichtung ist vorzugsweise ausgebildet, um Berechnungs-, Bestimmungs-, und Überprüfungsoperationen durchzuführen. Erfindungsgemäß werden durch die Datenverarbeitungseinrichtung die Lagen der Markereinrichtungen basierend auf den detektierten Signalen, die von den Markern ausgehen, berechnet. Vorzugsweise sind die Gegenstände (Körperstruktur oder Instrument), an denen die Markereinrichtungen angebracht sind, kalibriert. Dies bedeutet, dass die relativen Lagen zumindest zwischen Teilen des Gegenstandes und der Markereinrichtung, die an dem Gegenstand angebracht ist, bekannt sind und/oder insbesondere in der Datenverarbeitungseinrichtung abgespeichert sind, so dass insbesondere Anzeigesignale, die die Lagen der Gegenstände beschreiben, basierend auf den Lagen der Markereinrichtungen bestimmt werden können. Die Lagen der Markereinrichtungen werden bevorzugt relativ zu der Detektionseinrichtung berechnet, also in einem Bezugssystem, in dem die Detektionseinrichtung ruht. Natürlich können die Lagen auch in einem anderen Bezugssystem berechnet werden, z.B. in einem Bezugssystem, in dem eine der Markereinrichtungen ruht oder in dem der Patient ruht. Im Bezugssystem der Detektionseinrichtung (Kamerasystem) ruht der Patient, wenn sich die Detektionseinrichtung und der Patient nicht bewegt. Gemäß einer Ausführungsform der Erfindung wird von dieser Annahme zur Berechnung der Lage der Gegenstände ausgegangen. Gemäß einer anderen Ausführungsform wird beispielsweise am Patienten eine Markereinrichtung angebracht. Beispielhaft kann dies mit einer nicht invasiv angebrachten Markereinrichtung geschehen, die am Körper des Patienten befestigt wird und von der Detektionseinrichtung detektierbar ist. Ein Beispiel hierfür ist ein so genanntes "Headband", insbesondere ein "ENT-Headband", das mittels eines Gurtes an verschiedenen Körperstellen des Patienten angebracht werden kann, zum Beispiel an den Extremitäten. Auch kann eine Markereinrichtung an der Liegefläche, insbesondere Tisch, auf dem der Patient ruht, angebracht werden. Auch kann der Arzt als Bezugssystem gewählt werden, indem dieser beispielsweise mit einer Markereinrichtung, beispielsweise auch einem Kopfband versehen wird. Letztendlich kann auch der Behandlungsraum, insbesondere Operationsraum als Bezugssystem gewählt werden. Hierzu können beispielsweise Markereinrichtungen fest mit dem Raum, beispielsweise der Wand verbunden werden.

Weiter ist die Detektionseinrichtung vorzugsweise ausgebildet, Änderungen der Lagen der Markereinrichtungen zu bestimmen. Auch dies wird vorzugsweise im Bezugssystem der Detektionseinrichtung vorgenommen. Die Änderung wird basierend auf den berechneten Lagen bestimmt. Die Änderungen können zum Beispiel zwischen bestimmten Zeitabständen bestimmt werden. Dies bedeutet beispielsweise in bestimmten Zeitintervallen wird überprüft, ob sich die berechneten Lagen der Markereinrichtungen geändert haben. Auch kann dies so erfolgen, dass eine ständige Überprüfung einer Lageänderung durchgeführt wird und eine Änderung nur dann hinsichtlich ihres Umfangs bestimmt wird, wenn eine Lageänderung festgestellt wird. Die Änderungen der Lage der Markereinrichtungen können beispielsweise mit Vektoren in dem Bezugssystem der Detektionseinrichtung beschrieben werden.

Weiter wird erfindungsgemäß festgestellt, ob bestimmte Lageänderungen der Markereinrichtungen auf eine Relativbewegung der Markereinrichtungen zurückzuführen ist oder ob sie nur scheinbar durch eine Lageänderung der Detektionseinrichtung bewirkt wurde und/oder durch eine Lageänderung eines die Bewegung koppelnden Gegenstandes oder Objekts (im Folgenden Kopplungsgegenstand genannt) bewirkt wurde. Ein Beispiel für ein Objekt oder einen Kopplungsgegenstand ist der Patient oder eine Liege, auf der ein Patient liegt. Der Kopplungsgegenstand zeichnet sich dadurch aus, dass die Markereinrichtungen eine ortsfeste Beziehung bezüglich des Kopplungsgegenstandes haben, falls keine äußere Krafteinwirkung auf die Markereinrichtungen oder die damit verbundenen Gegenstände einwirkt.

Erfindungsgemäß wird nun überprüft, ob die bestimmten Änderungen der Lagen der Markereinrichtung durch eine Bewegung der Detektionseinrichtung oder des Kopplungsgegenstandes verursacht wurde. Bevorzugt wird hierzu überprüft, ob die bestimmten Änderungen der Lagen (jeder) der Markereinrichtungen durch eine (einzige) Transformation, insbesondere eine Transformationsmatrix darstellbar sind oder wären, die eine Lageänderung der Detektionseinrichtung oder eines Kopplungsgegenstandes beschreibt. Insbesondere wird überprüft, ob durch Translation und Rotation des Bezugssystems, in dem die Detektionseinrichtung ruht, die Lageänderungen beschreibbar sind. Weiter wird insbesondere überprüft, ob durch Rotation und/oder Translation eines Bezugssystems (ARF0), in dem die Markereinrichtungen vor der Änderung geruht haben, die Lageänderungen beschreibbar sind. Die Rotation und/oder Translation des Bezugssystems ARF0 wird ebenfalls insbesondere durch eine Transformation, insbesondere Transformationsmatrix beschrieben. Dies wird vorzugsweise mittels numerischer Verfahren durchgeführt, mit deren Hilfe insbesondere der Mittelpunkt der Drehung bestimmt werden kann. Der zuvor erwähnte Kopplungsgegenstand zeichnet sich insbesondere dadurch aus, dass er in dem Bezugssystem ARF0 ruht.

Im Falle einer Translationsbewegung aufgrund einer Lageänderung der Detektionseinrichtung oder des Kopplungsgegenstandes wird sich zeigen, dass die bestimmten Lageänderungen der Markereinrichtungen bzw. der diesen zugeordneten Bezugssysteme (ARF1, ARF2) durch denselben Vektor beschreibbar sind. Eine Drehbewegung des Kopplungsgegenstandes oder der Detektionseinrichtung lässt sich dadurch detektieren, dass ein Mittelpunkt der Drehung und eine Drehtransformation (Rotationsmatrix) bestimmt werden können, die für alle Markereinrichtungen bzw. die zugeordneten Bezugssysteme (ARF1 und ARF2) identisch sind.

Erfindungsgemäß ist die Datenverarbeitungseinrichtung vorzugsweise zur Überprüfung der Änderungen (Lageänderungen) ausgebildet. Dabei wird überprüft, ob die Lageänderungen (insbesondere alle Lageänderungen) durch eine (einzige) Transformation beschreibbar bzw. erklärbar sind oder wären. Ist dies der Fall, wird von einem positiven Überprüfungsergebnis ausgegangen. Diese Transformation wird hierin als "Überprüfungs-Transformation" bezeichnet. Die Überprüfungs-Transformation bewirkt insbesondere eine Translation oder eine Rotation oder eine Kombination aus Translation und Rotation.

Die Bestimmung einer Überprüfungs-Transformation kann erfindungsgemäß durchgeführt werden, muss aber nicht. Es genügt auch durch mathematische Verfahren festzustellen, ob die Lageänderungen durch eine (einzige) Transformation (Überprüfungs-Transformation) beschreibbar wären oder zumindest in einem vorbestimmten Umfang beschreibbar wären. Wären die Änderungen durch eine (einzige) Überprüfungs-Transformation (zumindest im Wesentlichen) beschreibbar, so geht man von einem positiven Überprüfungsergebnis aus. Ein positives Überprüfungsergebnis kann man auch ohne Bestimmung einer Überprüfungs-Transformation erhalten. Dies geschieht beispielsweise wie folgt. Man greift beispielsweise zwei der Markereinrichtungen heraus. Diese werden als erste und zweite Markereinrichtung bezeichnet. Eine relative Transformation wird bestimmt, die ein erstes Marker-Bezugssystem, in dem die erste Markereinrichtung ruht, in ein zweites Marker-Bezugssystem, in dem die zweite Markereinrichtung ruht, transformiert. Dann wird beobachtet und bestimmt, ob sich diese relative Transformation im Laufe der Zeit ändert und wenn ja, in welchem Umfang. Bleibt diese relative Transformation beispielsweise konstant, so geht man von einem positiven Überprüfungsergebnis aus. Das heißt selbst wenn sich die Lage der ersten Markereinrichtung und der zweiten Markereinrichtung z.B. in einem Kamera-Bezugssystem (in dem die Kamera ruht) ändert, ist diese Änderung auf eine gekoppelte Bewegung der ersten Markereinrichtung und der zweiten Markereinrichtung zurückzuführen, da die relative Transformation gleich bleibt. Dies hat zur Folge, dass in dem Kamera-Bezugssystem sowohl die Änderung der Lage der ersten Markereinrichtung als auch die Änderung der Lage der zweiten Markereinrichtung durch dieselbe Überprüfungs-Transformation beschreibbar wäre. Es ist also ein positives Überprüfungsergebnis gegeben, ohne dass eine konkrete Berechnung der Überprüfungs-Transformation notwendig ist. Die Bestimmung der vorgenannten relativen Transformation ist ausreichend. Der Umfang der Änderung der relativen Transformation lässt sich beispielsweise dadurch feststellen, dass eine relative Transformation zu einem ersten Zeitpunkt bestimmt wird und durch eine Matrix beschrieben wird. Diese Matrix wird mit der Inversen einer zweiten Matrix multipliziert, wobei die zweite Matrix die relative Transformation zu einem zweiten Zeitpunkt beschreibt. Ergibt sich als Ergebnis der Multiplikation eine Matrix, die der Einheitsmatrix ähnlich ist, so kann man davon ausgehen, dass sich die relative Transformation vom ersten Zeitpunkt zum zweiten Zeitpunkt wenig geändert hat. Um ein Maß für die Ähnlichkeit der relativen Transformationen zum ersten und zweiten Zeitpunkt zu erzielen, kann man die Differenz aus der vorgenannten Matrixmultiplikation und einer Einheitsmatrix bilden. Die Elemente der sich daraus ergebenden Matrix kann man dann auf ihren Betrag untersuchen. Beispielsweise kann man den Maximalwert der Elemente herausgreifen und mit einem Schwellenwert vergleichen. Ist dieser kleiner als der Schwellenwert, so geht man davon aus, dass die Änderung der relativen Transformation gering ist. Man hat also ein positives Überprüfungsergebnis. Alternativ kann man auch die Beträge der Elemente der Differenzmatrix addieren und mit einem Schwellenwert vergleichen. Weitere Verfahren wie Quadratbildung sind ebenfalls möglich. Die relative Transformation beschreibt vorzugsweise zumindest eine Translation und/oder Rotation.

Hat man mehr als zwei Markereinrichtungen, so kann man jeweils zwischen zwei Markereinrichtungen eine relative Transformation bestimmen und beispielsweise dann von einem positiven Überprüfungsergebnis ausgehen, wenn für z.B. eine bestimmte oder mehrere bestimmte oder insbesondere alle relativen Transformationen, die jeweils zwei Bezugssysteme zweier Markereinrichtungen verknüpfen, keine oder geringe Änderungen sich im Laufe der Zeit zeigen.

Eine Überprüfungs-Transformation kann auch zur Überprüfung konkret, insbesondere durch numerische Verfahren bestimmt werden und ist insbesondere dann gefunden, wenn eine Anwendung der Überprüfungs-Transformation in dem Bezugssystem der Detektionseinrichtung (CRF) dazu führt, dass die relativen Lagen (insbesondere jeder) der Markereinrichtungen relativ zum Detektionssystem vor den bestimmten Lageänderungen durch die Überprüfungs-Transformation in die neuen Lagen (insbesondere jeder) der Markereinrichtungen nach den bestimmten Lageänderungen übergeführt werden können. Beispielsweise kann zum Auffinden einer Überprüfungstransformation eine erste Transformation bestimmt werden, die die Lageänderung einer ersten Markereinrichtung beschreibt. Dann kann eine zweite Transformation bestimmt werden, die die Lageänderung einer zweiten Markereinrichtung beschreibt. Dann kann beispielsweise die erste Transformation als Überprüfungs-Transformation aufgefasst werden und mit der zweiten Transformation verglichen werden. Bei Übereinstimmung der Überprüfungs-Transformation (ersten Transformation) mit der zweiten Transformation oder einer Übereinstimmung zumindest im Wesentlichen kann dann ein positives Überprüfungsergebnis bestimmt werden. Entsprechendes gilt, falls eine Überprüfungs-Transformation gefunden werden kann, die die Lagen der Markereinrichtungen in einem gemeinsamen Bezugssystem ARF0 vor den bestimmten Änderungen (Lageänderungen) in die Lagen nach den bestimmten Änderungen (Lageänderungen) übergeführt werden kann. Anders ausgedrückt, werden die Lagen der Markereinrichtungen in dem Bezugssystem CRF oder ARF0 der Überprüfungs-Transformation unterzogen, so werden im Falle eines positiven Überprüfungsergebnisses die Lageänderungen der Markereinrichtungen in dem Bezugssystem CRF oder ARF0 kompensiert, d.h. die Lagen der Markereinrichtungen werden wieder in ihre Ausgangslagen (vor den Änderungen) zurückgeführt.

Die oben genannte Überprüfungs-Transformation wurde insbesondere im Hinblick auf jede der Markereinrichtungen betrachtet. Bei einer alternativen Ausführungsform kann eine vorbestimmte Gruppe aus den detektierten Markereinrichtungen herausgegriffen werden und eine gekoppelte Bewegung nur dieser Gruppe überprüft werden indem z.B. nur relative Transformationen zwischen Markereinrichtungen dieser Gruppe bestimmt und auf ihre Veränderung mit der Zeit untersucht werden. Beispielsweise kann es sich bei dieser Gruppe von Markereinrichtungen um solche handeln, die fest mit dem Patienten verbunden sind, während auch noch weitere Markereinrichtungen von der Detektionseinrichtung erfasst werden, die nicht fest mit dem Patienten verbunden werden und bezüglich derer das Erfassen einer gekoppelten Bewegung nicht von Interesse ist. Die Erfindung umfasst also insbesondere auch die Ausführungsform, wonach überprüft wird, ob die Änderung der Lage einer bestimmten Gruppe der detektierten Markereinrichtungen durch eine Überprüfungs-Transformation beschreibbar ist oder beschreibbar wäre.

Oben wurde die Anwendung der Überprüfurigs-Transformation so beschrieben, dass sie in einem Bezugssystem (CAF oder ARF0) auf die Lagen (die beispielsweise durch Koordinaten beschrieben werden) angewendet wird. Alternativ kann man sich dies natürlich auch so vorstellen, dass die Überprüfungs-Transformation auf das jeweilige Bezugssystem (CAF oder ARF0) angewendet wird, so dass sich (beispielsweise durch eine Drehung des Bezugssystems) wieder dieselben Koordinaten für die Markereinrichtungen ergeben, wie sie vor den bestimmten Lageänderungen galten.

Die Bestimmung der Überprüfungs-Transformation kann mit Unsicherheiten verbunden sein oder die bestimmten Änderungen der Lagen der Markereinrichtungen können wesentlich aber nicht ausschließlich durch eine Bewegung der Detektionseinrichtung oder des Kopplungsgegenstandes verursacht worden sein. Das heißt, es kann auch einen gewissen Anteil an einer Relativbewegung zwischen den Markereinrichtungen geben. Wie zuvor ausgeführt, wird ein positives Überprüfungsergebnis festgestellt, wenn die Lageänderungen auf eine Bewegung der Detektionseinrichtung und/oder des Kopplungsgegenstandes zurückzuführen sind. Vorzugsweise wird ein positives Überprüfungsergebnis aus den vorgenannten Gründen auch dann festgestellt, wenn (sämtliche) Lageänderungen (zumindest) wesentlich durch eine (einzige) Überprüfungs-Transformation beschreibbar sind. Um das Wort "wesentlich" für die Datenverarbeitung mittels der Datenverarbeitungseinrichtung zu quantifizieren, wird vorzugsweise eine Überprüfungs-Transformation bestimmt, nach deren Anwendung es zu den kleinstmöglichen Abweichungen zwischen den Lagen der Markereinrichtungen vor den bestimmten Änderungen und den Lagen der Markereinrichtungen nach den bestimmten Änderungen kommt. Zur Quantifizierung wird beispielsweise diejenige Markereinrichtung herausgegriffen, für die sich die größte Abweichung ergab. Eine erste Abweichung der ersten Lage (Lage vor der bestimmten Änderung) von der zweiten Lage (Lage nach der bestimmten Änderung und nach Anwendung der Überprüfungs-Transformation zur Kompensation der Lageänderung) kann beispielsweise durch den Abstand zwischen der ersten und zweiten Lage quantifiziert werden. Weiter kann eine zweite Abweichung als Differenz der ersten Lage (Lage vor der bestimmten Lageänderung) von einer dritten Lage (Lage nach der bestimmten Lageänderung und ohne Anwendung einer Überprüfungs-Transformation) berechnet werden. Die erste Abweichung kann dann zur zweiten Abweichung ins Verhältnis gesetzt werden. Ist dieses Verhältnis kleiner als ein vorbestimmter Prozentsatz, so wird das Überprüfungsergebnis als positiv angesehen. Der vorbestimmte Prozentsatz kann z.B. weniger als 50 % oder 25 % oder weniger als 10 % oder weniger als 5 % oder weniger als 1 % betragen. Ist das Kriterium "kleiner als ein vorbestimmter Prozentsatz" erfüllt, so wird davon ausgegangen, dass die Änderungen der Lagen jeder der Markereinrichtungen zumindest in einem vorbestimmten Umfang durch die Überprüfungs-Transformation beschreibbar sind.

Alternativ oder zusätzlich zu der vorgenannten Verhältnisbildung kann das genannte Kriterium "wesentlich" auch durch absolute Schwellenwerte qualifiziert werden. Falls beispielsweise die erste Abweichung kleiner als ein vorgegebener Schwellenwert ist, geht man davon aus, dass das Überprüfungsergebnis positiv ist, also die Lageänderung wesentlich durch die Überprüfungs-Transformation beschreibbar ist. Alternativ oder zusätzlich kann man gemäß einer weiteren Ausführungsform dann von einem positiven Überprüfungsergebnis ausgehen, wenn die zweite Abweichung oberhalb eines bestimmten Schwellenwertes ist. Insbesondere können mehrere erste Schwellenwerte für die erste Abweichung und mehrere zweite Schwellenwerte für die zweite Abweichung im vorgenannten Sinne festgelegt werden, wobei jeweils ein erster Schwellenwert einem zweiten Schwellenwert zugeordnet ist (z.B. in Gestalt einer Tabelle) und von einem positiven Überprüfungsergebnis dann ausgegangen wird, wenn der erste Schwellenwert durch die erste Abweichung unterschritten wird und der zweite Schwellenwert durch die zweite Abweichung überschritten wird.

Vorzugsweise erfolgt die Bestimmung von Anzeigesignalen, die die Lagen der Markereinrichtungen und/oder der damit verbundenen Gegenstände relativ zu einem Anzeigebezugssystem zeigen in Abhängigkeit von dem Überprüfungsergebnis. Dies bedeutet insbesondere, dass im Falle eines positiven Überprüfungsergebnisses die detektierten Lageänderungen nicht weiter verarbeitet werden und somit das bisherige Anzeigesignal, das an die Anzeigeeinrichtung gesendet wird, beibehalten wird.

Die Bestimmung von Anzeigesignalen in Abhängigkeit von dem Überprüfungsergebnis schließt nicht aus, dass die Anzeigesignale auch noch in Abhängigkeit von weiteren (optionalen) Bedingungen bestimmt werden. Zu diesen Bedingungen zählt insbesondere, ob die vor der Lageänderung angezeigten Gegenstände auch nach Darstellung dieser Lageänderung noch auf einer Anzeigeeinrichtung (beispielsweise Bildschirm) zu sehen wären. Eine weitere optionale Bedingung, in deren Abhängigkeit die Anzeigesignale bestimmt werden können, stellt beispielsweise auch die Geschwindigkeit der Lageänderung dar. Die Geschwindigkeit kann beispielsweise aus dem Umfang der Lageänderung (also Abstand zwischen oben erwähnter erster Lage und dritter Lage) geteilt durch die Zeit während der die Lageänderung erfolgte, berechnet werden. Beispielsweise können nur Lageänderungen angezeigt werden, die mit einer Geschwindigkeit erfolgen, die oberhalb oder unterhalb eines bestimmten Geschwindigkeits-Schwellenwertes liegt. Alternativ oder zusätzlich kann die Anzeige von Lageänderungen unterdrückt werden, wenn die Lageänderungen mit einer Geschwindigkeit erfolgen, die oberhalb eines ersten Geschwindigkeitsschwellenwertes und/oder unterhalb eines zweiten bestimmten Geschwindigkeits-Schwellenwertes liegt. Die vorgenannten Bedingungen können dazu genutzt werden, dass beispielsweise für einen Operateur nur schnelle Lageänderungen angezeigt werden, wenn nur diese für den Operateur interessant sind. Alternativ können gerade die schnellen Lageänderungen unterdrückt werden, falls diese beispielsweise eine den Operateur störende, durch Vibrationen bedingte Bewegung darstellen.

Erfindungsgemäß wird also insbesondere in Abhängigkeit von dem Überprüfungsergebnis und optional auch von weiteren Bedingungen durch die Anzeigeeinrichtung keine Änderung der Lagen angezeigt. Dies wird mittels der erfindungsgemäßen Datenverarbeitungseinrichtung beispielsweise so umgesetzt, indem die Anzeigesignale unverändert belassen werden. Alternativ wird der Anzeigebereich oder das Bezugssystem der Anzeige in dem die Markereinrichtungen und/oder die damit verbundenen Gegenstände angezeigt werden, der bestimmten Überprüfungs-Transformation so unterzogen, dass sich keine Änderung auf der Anzeige zeigt oder nur diejenigen Änderungen gezeigt werden, die durch die Anwendung der Überprüfungs-Transformation nicht kompensiert werden. Letzteres hat zur Folge, dass für den Fall einer Überlagerung aus einer Relativbewegung zwischen den Markereinrichtungen und einer Bewegung der Detektionseinrichtung und/oder des Kopplungsgegenstandes (nur) die Relativbewegung gezeigt wird, während die Änderungen aufgrund der Bewegung der Detektionseinrichtung und/oder des Kopplungsgegenstandes (Kopplungsobjekts) nicht gezeigt werden.

Es erfolgt also bei positivem Überprüfungsergebnis eine Änderung der üblichen Verarbeitung der Detektionssignale. Gemäß einer Ausführungsform, wird aber auch im Falle eines positiven Überprüfungsergebnisses die übliche Darstellung beibehalten. Allerdings nur so lange, wie vorbestimmte Gegenstände, die beispielsweise bisher durch die Anzeigeeinrichtung dargestellt wurden, weiterhin auf der Anzeigeeinrichtung sichtbar bleiben.

Es kann beispielsweise auftreten, dass eine Drift nicht vollständig durch das erfindungsgemäße Verfahren kompensiert wird. Die Ursache kann in numerischen Ungenauigkeiten liegen oder in den vorgenannten Bedingungen, falls nur Lageänderungen mit Geschwindigkeiten, die in einem vorbestimmten Geschwindigkeitsbereich liegen, kompensiert werden sollen. Eine andere Ursache dafür, dass die vorbestimmten Gegenstände den Anzeigebereich verlassen, kann an Absolutbewegungen (aller) dargestellter Gegenstände in eine Richtung liegen. Für die vorgenannten Fälle kann es sich also ergeben, dass sich zumindest einer der angezeigten Gegenstände aus dem sichtbaren Bereich bewegt, also z.B. nur noch zum Teil dargestellt wird. Dann erfolgt vorzugsweise oder optional eine erneute Ausrichtung der dargestellten Gegenstände, insbesondere Zentrierung, so dass alle Gegenstände vollständig von der Anzeigeeinrichtung dargestellt werden. Vorzugsweise ist hierzu die Datenverarbeitungseinrichtung so ausgebildet, dass sie einen gleichen minimalen Abstand der dargestellten Gegenstände zum Rand des angezeigten Bereiches einstellt. Anders ausgedrückt, durch die Neuzentrierung ist der minimale Abstand eines jeden Gegenstandes zum Rand des Anzeigebereichs vorzugsweise gleich. Die erneute Ausrichtung der dargestellten Gegenstände kann manuell von dem Bediener bewirkt werden oder auch automatisch erfolgen, wobei insbesondere im letzteren Fall vorzugsweise der Bediener durch ein Warnsignal auf die erneute Ausrichtung hingewiesen wird.

Gemäß einer weiteren Ausführungsform der Erfindung, ist die Datenverarbeitungseinrichtung ausgebildet, die Überprüfungs-Transformation wie folgt zu bestimmen. Für jeden detektierten Gegenstand, zumindest für jeden auf der Anzeigeeinrichtung dargestellten Gegenstand wird eine Transformation berechnet, die hierin als Änderungstransformation bezeichnet wird. Die Änderungstransformation transformiert die erste Lage (vor der Änderung) in die dritte Lage (nach der Änderung) für jeden Gegenstand. Die Änderungstransformation erfolgt vorzugsweise im Bezugssystem ARFO, in dem die Bezugssysteme der (dargestellten) Gegenstände vor der Änderung ruhten. In einem nächsten Schritt werden dann die Änderungenstransformationen für die einzelnen (dargestellten) Gegenstände miteinander verglichen. Falls die Änderungstransformationen gleich sind, wird ein positives Überprüfungsergebnis festgestellt. Vorzugsweise wird auch ein positives Überprüfungsergebnis festgestellt, falls die Änderungstransformationen sich in einem vorbestimmten Umfang ähneln. Letzteres bedeutet insbesondere, dass bei Anwendung einer der bestimmten Änderungstransformationen auf die erste Lage aller dargestellten Gegenstände sich eine Lage jeweils ergibt, die für jeden Gegenstand nur im geringen Umfang von der dritten Lage (nach Lageänderung) abweicht. Ein geringer Umfang bedeutet hierbei insbesondere, dass das Verhältnis der Abweichung relativ zu dem Umfang der Lageänderung kleiner als eine vorbestimmte Prozentzahl ist. Die vorbestimmte Prozentzahl beträgt beispielsweise 25 % oder 10 % oder 5 % oder 1 %.

Gemäß einer Ausführungsform bestimmt die Datenverarbeitungseinrichtung aus den für die jeweiligen dargestellten Gegenstände bestimmten Änderungstransformationen die Überprüfungs-Transformation z.B. durch Mitteln der Änderungstransformationen oder indem eine der Änderungstransformationen herausgegriffen wird.

Wie bereits zuvor erwähnt, kann eine Geschwindigkeit der Änderung berechnet werden. Dazu wird beispielsweise für einen Gegenstand der Abstand zwischen der dritten Lage (nach der Änderung) und der ersten Lage (vor der Änderung) berechnet und durch die Zeit geteilt, während der diese Lageänderung erfolgte. Ist die Geschwindigkeit unterhalb eines vorgegebenen Schwellenwertes, so wird im Falle eines positiven Überprüfungsergebnisses vorzugsweise keine Änderung der Lagen angezeigt. Liegt die Geschwindigkeit über dem Schwellenbetrag, so wird vorzugsweise die detektierte Lageänderung angezeigt. Der Schwellenwert für die Geschwindigkeit liegt beispielsweise über 0,1 mm/s oder 1 mm/s oder 3 mm/s oder 1 cm/s und/oder vorzugsweise unter 10 cm/s oder 3 cm/s oder 1 cm/s oder 1 mm/s. Auf diese Art und Weise kann insbesondere eine langsame Drift der angezeigten Gegenstände eliminiert werden.

Bei einer Ausführungsform erfolgt vorzugsweise im Falle einer Geschwindigkeit über dem Schwellenwert auch dann keine Anzeige der Lageänderung, wenn ansonsten, also im Falle der Anzeige der Lageänderung, zumindest einer der dargestellten Gegenstände den angezeigten Bereich zumindest zum Teil verlassen würde.

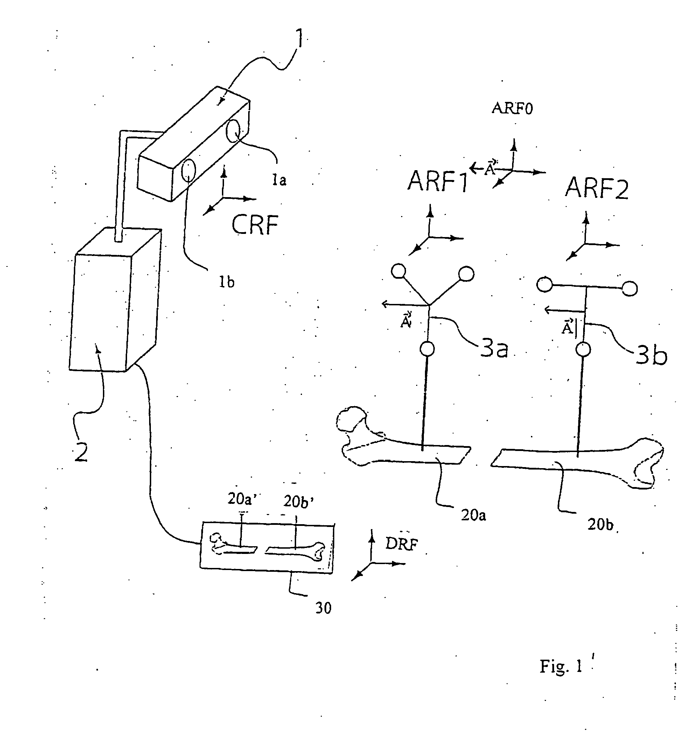

Figur 1 zeigt eine Ausführungsform eines erfindungsgemäßen Markernavigationssystems.

In Figur 1 ist das Beispiel gezeigt, dass an zwei relativ zueinander beweglichen Knochenteilen 20a und 20b jeweils ein Referenzstern 3a und 3b angebracht ist. Der Referenzstern ist ein Beispiel für eine Markereinrichtung und umfasst drei Markerkugeln, die in charakteristischen relativen Lagen zueinander angeordnet sind.

Weiter ist eine Kamera 1 vorgesehen, die als Detektionseinrichtung dient. Die Kamera 1 umfasst Detektionselemente 1a und 1b. Die Kamera 1 ist über eine Datensignalleitung mit einer Datenverarbeitungseinrichtung 2, beispielsweise einem Computer verbunden. Der Kamera 1 ist ein Kamerabezugssystem (CRF) zugeordnet. Dem Referenzstern 3a ist ein mit ARF1 (Array Reference Frame) bezeichnetes Bezugssystem zugeordnet. Dem Referenzstern 3b ist ein mit ARF2 bezeichnetes Bezugssystem zugeordnet. Die Kamera ruht in dem Bezugssystem CRF. Der Referenzstern 3a ruht in dem Bezugssystem ARF1. Der Referenzstern 3b ruht in dem Bezugssystem ARF2.

Bevorzugt werden zwei Detektionselemente 1a und 1b vorgesehen, um analog zum menschlichen räumlichen Sehen, die relative Lage der Referenzsterne 3a und 3b relativ zur Detektionseinrichtung 1 aus dem Detektionssignal mithilfe der Datenverarbeitungseinrichtung 2 berechnen zu können.

Bei der Verwendung des erfindungsgemäßen Markernavigationssystems kann sich nun folgende Situation ergeben. Die Referenzsterne 3a und 3b ändern ihre Lage nicht, aber die Kamera 1 ändert ihre Lage. Wird diese Lageänderung der Kamera 1 nicht berücksichtigt, so verändert sich auf einer Anzeige 30 die Anzeige der Knochenteile 20a' und 20b', obwohl diese realiter nicht bewegt wurden. Eine andere und vergleichbare Situation ergibt sich dann, wenn der Patient beispielsweise auf einer Unterlage (Kopplungsgegenstand bzw. Kopplungsobjekt) liegend bewegt wird. Diese Bewegungen können dazu führen, dass die Darstellung der Knochenteile 20a' und 20b' aus dem angezeigten Bereich verschwinden. Auch letzteres ist möglicherweise nicht erwünscht.

Erfindungsgemäß wird dann auf eine Bewegung der Kamera 1 und/oder eines Kopplungsobjekts bzw. Kopplungsgegenstandes (also beispielsweise des Patienten oder einer Liege) geschlossen, falls die detektierte Bewegung durch eine Bewegung der Kamera 1 und/oder eines Kopplungsobjekts bzw. Kopplungsgegenstandes (beispielsweise des Patienten) erklären lässt. Das in Figur 1 gezeigte Bezugssystem ARF0 ist mit dem Kopplungsgegenstand verbunden.

# EP 1 923 015 B1

Wird beispielsweise in Figur 1 eine Verschiebung des Referenzsterns 3a und 3b jeweils um einen gleichen Vektor A detektiert, so ist dies durch eine Verschiebung des Patienten oder beispielsweise der Auflagefläche (auf der der Patient liegt) erklärbar. In diesem Fall kann erfindungsgemäß deshalb eine Anzeige der Verschiebung unterbunden werden. Dies wird dadurch bewerkstelligt, indem beispielsweise die von der Datenverarbeitungseinrichtung 2 an die Anzeige 30 übermittelten Anzeigesignale trotz der detektierten Änderung nicht geändert werden. Alternativ kann das Anzeigebezugssystem CRF (Camera Reference Frame) ebenfalls um den Vektor A verschoben werden, so dass sich keine Änderung der Position der Knochen 20a' und 20b' im Bezugssystem CRF ergibt und somit auch nicht auf der Anzeige 30 zeigt.

Vorzugsweise wird aus der detektierten Änderung der Referenzsterne 3a und 3b durch numerische Verfahren ermittelt, ob diese durch eine Transformationsmatrix beschreibbar ist, die durch eine Translation oder Rotation des Bezugssystems CRF oder eines Bezugssystems ARF0 beschreibbar ist. In dem Bezugssystem ARF0 ruhen die Bezugssysteme der Referenzsterne, also hier das Bezugssystem ARF 1 und ARF2 vor der Änderung.

Beispielsweise kann eine erfindungsgemäße Überprüfung, ob die Lageänderungen zweier Markereinrichtungen durch eine (einzige) Überprüfungstransformation beschreibbar wären, wie folgt durchgeführt werden. In einer konkreten Implementierung werden die Positionen zweier Markeranordnungen A und B in Kamerakoordinaten erfasst, wobei hier die Positionen durch Koordinatentransformationen $M_A$ bzw. $M_B$ vom Bezugssystem der Kamera in das jeweilige Bezugssystem A bzw. B ausgedrückt werden. Im Wesentlichen können diese durch (4, 4)-Matrizen dargestellt werden. Diese enthalten i.A. einen Rotations- und einen Translationsanteil.

Zu einem initialen Zeitpunkt $t_0$ werden die Koordinatentransformationen $M_A$ $(t_0)$ und $M_B$ $(t_0)$ erfasst und gespeichert. Zusätzlich wird eine initiale relative oordinatentransformation $M_{BA,last}$ gespeichert. Zu aufeinander folgenden Zeitpunkten $t_i$ werden dann die Koordinatentransformationen $M_A$ $(t_i)$ und $M_B$ $(t_i)$ erneut erfasst und daraus die relative Koordinatentransformation $M_{BA}(t_i)$ ermittelt.

Zunächst wird nun überprüft, ob eine **gekoppelte** Bewegung der Anordnungen A und B vorliegt, indem eine "Differenzmatrix" aus den ermittelten relativen Koordinatentransformationen gebildet wird:

$$\Delta M_{BA}(t_i) \quad = \quad M_{BA}(t_i) \quad * \quad (M_{BA,last})^{-1} - 1_4 \qquad \text{(Letzteres ist die vierdimensionale Einheitsmatrix)}$$

Das Kriterium für eine gekoppelte Bewegung und damit für ein positives Überprüfungsergebnis ist, dass der Unterschied zwischen aufeinanderfolgenden relativen Positionen sehr klein ist. Mathematisch lässt sich dies beispielsweise so erfassen, dass die Maximumnorm der errechneten Differenzmatrix kleiner sein muss als ein vorgegebener absoluter Schwellenwert:

$$\left\| \Delta M_{BA}(t_i) \right\|_{max} < \text{ Schwellenwert, d.h. das Element der Matrix mit dem höchsten Wert muss kleiner als der Schwellenwert sein.}$$

Als Abschluss dieses Schrittes wird der Matrix $M_{BA,last}$ der Wert von $M_{BA}(t_i)$ zugewiesen, so dass zum Zeitpunkt $t_{i+1}$ wieder auf die vorherige Relativposition zurückgegriffen werden kann.

Wurde eine gekoppelte Bewegung erkannt, ist also das Überprüfungsergebnis positiv, so wird diese in der Anzeige der Markereinrichtungen unterdrückt, d.h. keine der Markereinrichtungen bzw. keiner der damit verbundenen Körper wird in der Anzeige bewegt.

Liegt keine gekoppelte Bewegung vor, so wird in der Anzeige jede der Markereinrichtungen A und B so bewegt, wie sich diese Bewegung im Kamerasystem darstellt. Hierzu wird in analoger Weise wie oben die relative Veränderung der Position für jede Markeranordnung einzeln errechnet, durch Vergleich mit der Position aus dem vorherigen Zeitschritt. Lag zuvor eine gekoppelte Bewegung vor, so wurde die Position nicht neu gespeichert, um die Anzeige nur der ungekoppelten Bewegungen zu ermöglichen.

**Patentansprüche**

1. Markernavigationssystem zur Erfassung und Darstellung der Lage von Markereinrichtungen, die an Gegenständen, insbesondere an Körperstrukturen und/oder medizinischen Instrumenten angebracht sind,

mit einer Detektionseinrichtung (1) zum Detektieren von Signalen, die von den Markereinrichtungen (3a, 3b) ausgehen;
mit einer Datenverarbeitungseinrichtung (2)

zum Berechnen der Lagen der Markereinrichtungen (3a, 3b) basierend auf den detektierten Signalen;
zum Bestimmen von Änderungen der Lagen der Markereinrichtungen basierend auf den berechneten Lagen;
zur Überprüfung basierend auf den bestimmten Änderungen, ob die Änderungen der Lage der Markereinrichtungen (3a, 3b) durch eine und insbesondere eine einzige Überprüfungs-Transformation beschreibbar sind oder wären, die die Lagen der Markereinrichtungen (3a, 3b) vor der Änderung in die Lagen der Markereinrichtungen (3a, 3b) nach der Änderung zumindest in einem vorbestimmten Umfang transformiert oder transformieren würde;
zur Bestimmung von Anzeigesignalen, die die Lagen der Markereinrichtungen (3a, 3b) und/oder der mit den Markereinrichtungen verbundenen Gegenstände darstellen, in Abhängigkeit von dem Überprüfungsergebnis;

mit einer Anzeigeeinrichtung (30)

zur Anzeige der Lagen entsprechend den Anzeigesignalen.

2. Markernavigationssystem nach Anspruch 1, bei welchem ein positives Überprüfungsergebnis dann bestimmt wird, wenn sich mindestens eine relative Transformation, die eine Transformation von einem ersten Marker-Bezugssystem, in dem eine erste der Markereinrichtungen ruht, zu einem zweiten Marker-Bezugssystem, in dem eine zweite der Markereinrichtungen ruht, durchführt, mit der Zeit nicht oder weniger als in einem vorbestimmten Umfang ändert.

3. Markernavigationssystem nach Anspruch 1 oder 2, bei welchem im Falle eines positiven Überprüfungsergebnis die bestimmten Änderungen nicht oder nur teilweise angezeigt werden.

4. Markernavigationssystem nach einem der Ansprüche 1 bis 3, bei welchem die Datenverarbeitungseinrichtung (2) so ausgebildet ist, dass sie im Falle eines positiven Überprüfungsergebnisses die Anzeigesignale beibehält, die die bestimmten Änderungen nicht wiederspiegeln.

5. Markernavigationssystem nach einem der Ansprüche 1 bis 4, bei welchem die Datenverarbeitungseinrichtung (2) so ausgebildet ist, dass im Falle eines positiven Überprüfungsergebnisses die sich nach der Änderung ergebenden Lagen der Markereinrichtung einer Transformation unterzogen werden, die die Änderung der Lage zumindest zum Teil rückgängig macht, und die Anzeigesignale basierend auf den transformierten Lagen berechnet werden.

6. Markernavigationssystem nach Anspruch 5, bei welchem die Transformation der Überprüfungs-Transformation entspricht oder aus dieser berechnet wird.

7. Markernavigationssystem nach einem der Ansprüche 1 bis 3, bei welchem die Datenverarbeitungseinrichtung so ausgebildet ist, dass sie die Anzeigesignale so unverändert lässt, dass durch die Anzeigeeinrichtung keine Änderung der Lagen angezeigt wird oder die bestimmten Änderungen nur zu einem Teil angezeigt werden und/oder die geänderten und bestimmten Lagen vor der Bestimmung der Anzeigesignale im Falle eines positiven Überprüfungsergebnis der Überprüfungs-Transformation so unterzogen werden, dass die Änderungen zumindest zum Teil rückgängig gemacht werden.

8. Markernavigationssystem nach einem der Ansprüche 1 bis 7, bei welchem die Datenverarbeitungseinrichtung ausgebildet ist, die Überprüfungs-Transformation basierend auf der Annahme zu bestimmen,

dass die bestimmten Änderungen durch eine Drehung und/oder Translation der Detektionseinrichtung bewirkt wurden und/oder
dass die bestimmten Änderungen durch Drehung und/oder Translation eines Objekts bewirkt wurden, relativ zu dem die Markereinrichtungen ortsfest sind.

9. Markernavigationssystem nach einem der Ansprüche 1 bis 8, bei welchem die Datenverarbeitungseinrichtung (2) ausgebildet ist:

die Überprüfungs-Transformation zu bestimmen, indem jeweils eine Änderungstransformation, die die Änderung der Lage von vor der Änderung zu nach der Änderung beschreibt, für jede der Markereinrichtungen berechnet; die berechneten Änderungstransformationen miteinander zu vergleichen; und falls sich alle Änderungstransformationen zumindest in einem vorbestimmten Umfang ähneln, ein positives Überprüfungsergebnis festzustellen.

**10.** Markernavigationssystem nach Anspruch 9, bei welchem die Datenverarbeitungseinrichtung (2) ausgebildet ist, aus den Änderungstransformationen die Überprüfungs-Transformation zu bestimmen.

**11.** Markernavigationssystem nach einem der vorhergehenden Ansprüche, bei welchem im Falle eines positiven Überprüfungsergebnisses die Geschwindigkeit der Änderungen berechnet werden und, falls die berechnete Geschwindigkeit kleiner oder größer als eine vorbestimmte Geschwindigkeit ist, die Anzeigesignale unverändert gelassen werden oder die Anzeigesignale so bestimmt werden, dass die bestimmten Änderungen nicht oder nur zum Teil angezeigt werden.

**12.** Markernavigationssystem nach Anspruch 11, bei welchem, falls die berechnete Geschwindigkeit größer oder gleich der vorbestimmten Geschwindigkeit ist, die bestimmten Änderungen nicht oder nur zum Teil angezeigt werden, falls bei Anzeige der Lagen, die sich gemäß den bestimmten Änderungen geändert haben, nicht mehr alle zuvor dargestellten Lagen von der Anzeigeeinrichtung angezeigt werden.

**13.** Verfahren zum Erfassen und Darstellen der Lage von Markereinrichtungen mittels eines Markernavigationssystems, wobei die Markereinrichtungen an Gegenständen, insbesondere an Körperstrukturen und/oder medizinischen Instrumenten angebracht sind und wobei das Verfahren die folgenden Schritte umfasst:

Signale, die von den Markereinrichtungen ausgehen, werden detektiert;
die Lagen der Markereinrichtungen (3a, 3b) werden basierend auf den detektierten Signalen berechnet;
Änderungen der Lagen der Markereinrichtungen werden basierend auf den berechneten Lagen bestimmt;
basierend auf den bestimmten Änderungen wird überprüft, ob die Änderungen der Lage jeder der Markereinrichtungen (3a, 3b) durch eine Überprüfungs-Transformation beschreibbar ist, die für alle Änderungen gleich ist, und die die Lagen der Markereinrichtungen (3a, 3b) vor der Änderung in die Lagen der Markereinrichtungen (3a, 3b) nach der Änderung zumindest in einem vorbestimmten Umfang transformiert;
in Abhängigkeit von dem Überprüfungsergebnis werden Anzeigesignale bestimmt, die die Lagen der Markereinrichtungen (3a, 3b) und/oder der mit den Markereinrichtungen verbundenen Gegenstände darstellen;
die Lagen werden entsprechend den Anzeigesignalen angezeigt.

**Claims**

**1.** A marker navigation system for detecting and displaying the location of marker means which are attached to objects, in particular body structures and/or medical instruments, comprising:

a detection means (1) for detecting signals emitted from the marker means (3a, 3b);
a data processing means (2) for:

calculating the locations of the marker means (3a, 3b) on the basis of the detected signals;
determining changes in the locations of the marker means on the basis of the calculated locations;
checking, on the basis of the determined changes, whether the changes in the location of the marker means (3a, 3b) can or could be described by a checking transformation and in particular by a single checking transformation which at least to a predetermined extent transforms or would transform the locations of the marker means (3a, 3b) before the change into the locations of the marker means (3a, 3b) after the change;
determining display signals which display the locations of the marker means (3a, 3b) and/or of the objects connected to the marker means, as a function of the checking result;

a display means (30) for:

displaying the locations in accordance with the display signals.

**2.** The marker navigation system according to claim 1, wherein a positive checking result is determined when at least one relative transformation which transforms a transformation from a first marker reference frame in which a first of the marker means lies into a second marker reference frame in which a second of the marker means lies, does not change or at least changes less than to a predetermined extent over time.

3. The marker navigation system according to claim 1 or 2, wherein if a checking result is positive, the determined changes are not displayed or are only partially displayed.

4. The marker navigation system according to any one of claims 1 to 3, wherein the data processing means (2) is designed such that if a checking result is positive, it retains the display signals which do not reflect the determined changes.

5. The marker navigation system according to any one of claims 1 to 4, wherein the data processing means (2) is designed such that if a checking result is positive, the locations of the marker means after the change are subjected to a transformation which at least partially reverses the change in the location, and the display signals are calculated on the basis of the transformed locations.

6. The marker navigation system according to claim 5, wherein the transformation corresponds to or is calculated from the checking transformation.

7. The marker navigation system according to any one of claims 1 to 3, wherein the data processing means is designed such that it leaves the display signals unchanged, such that the display means does not display any change in the locations or the determined changes are only partially displayed and/or the changed and determined locations are subjected to the checking transformation before determining the display signals if a checking result is positive, such that the changes are at least partially reversed.

8. The marker navigation system according to any one of claims 1 to 7, wherein the data processing means is designed to determine the checking transformation on the basis of the assumption:

that the determined changes have been caused by rotating and/or translating the detection means; and/or
that the determined changes have been caused by rotating and/or translating an object, relative to which the marker means are stationary.

9. The marker navigation system according to any one of claims 1 to 8, wherein the data processing means (2) is designed to:

determine the checking transformation by calculating for each of the marker means a change transformation which describes the change in the location from before the change to after the change;
compare the calculated change transformations with each other; and
establish a positive checking result if all the change transformations are similar to at least a predetermined extent.

10. The marker navigation system according to claim 9, wherein the data processing means (2) is designed to determine the checking transformation from the change transformations.

11. The marker navigation system according to any one of the preceding claims, wherein if a checking result is positive, the speed of the changes is calculated; and
if the calculated speed is smaller or greater than a predetermined speed, the display signals are left unchanged or the display signals are determined such that the determined changes are not displayed or are only partially displayed.

12. The marker navigation system according to claim 11, wherein if the calculated speed is greater than or equal to the predetermined speed, the determined changes are not displayed or are only partially displayed, if - when the locations which have changed in accordance with the determined changes are displayed - no longer all the previously displayed locations are displayed by the display means.

13. A method for detecting and displaying the location of marker means by means of a marker navigation system, wherein the marker means are attached to objects, in particular body structures and/or medical instruments, and wherein the method comprises the steps of:

detecting signals emitted from the marker means;

calculating the locations of the marker means (3a, 3b) on the basis of the detected signals; determining changes in the locations of the marker means on the basis of the calculated locations;

checking, on the basis of the determined changes, whether the changes in the location of each of the marker means (3a, 3b) can be described by the checking transformation which is the same for all the changes and which at least to a predetermined extent transforms the locations of the marker means (3a, 3b) before the change into the locations of the marker means (3a, 3b) after the change;

determining, as a function of the checking result, display signals which display the locations of the marker means (3a, 3b) and/or the objects connected to the marker means; displaying the locations in accordance with the display signals.

## Revendications

1. Système de navigation à marqueurs pour détecter et représenter la position de dispositifs de marquage qui sont fixés à des objets, en particulier à des structures corporelles et/ou à des instruments médicaux, comprenant

   un dispositif de détection (1) pour détecter des signaux qui partent des dispositifs de marquage (3a, 3b) ;
   un dispositif de traitement de données (2)

   pour calculer les positions des dispositifs de marquage (3a, 3b) sur la base des signaux détectés ;
   pour déterminer des changements des positions des dispositifs de marquage sur la base des positions calculées ;
   pour vérifier sur la base des changements déterminés si les changements de la position des dispositifs de marquage (3a, 3b) peuvent ou pourraient être décrits par une, en particulier une seule transformation de vérification qui transforme ou transformerait les positions des dispositifs de marquage (3a, 3b) avant le changement vers les positions des dispositifs de marquage (3a, 3b) après le changement, au moins dans une mesure prédéterminée ;
   pour déterminer des signaux d'affichage qui représentent les positions des dispositifs de marquage (3a, 3b) et/ou les objets reliés aux dispositifs de marquage, en fonction du résultat de vérification ;

   un dispositif d'affichage (30)

   pour afficher les positions selon les signaux d'affichage.

2. Système de navigation à marqueurs selon la revendication 1, dans lequel un résultat de vérification positif est déterminé si au moins une transformation relative, qui effectue une transformation d'un premier système de référence de marqueurs dans lequel se trouve un premier des dispositifs de marquage en un deuxième système de référence de marqueurs dans lequel se trouve un deuxième des dispositifs de marquage, ne change pas dans le temps ou change moins que dans une mesure prédéterminée.

3. Système de navigation à marqueurs selon la revendication 1 ou 2, dans lequel, dans le cas d'un résultat de vérification positif, les changements déterminés ne sont pas affichés ou sont affichés en partie seulement.

4. Système de navigation à marqueurs selon l'une quelconque des revendications 1 à 3, dans lequel le dispositif de traitement de données (2) est réalisé de telle sorte que dans le cas d'un résultat de vérification positif, il conserve les signaux d'affichage qui ne reflètent pas les changements déterminés.

5. Système de navigation à marqueurs selon l'une quelconque des revendications 1 à 4, dans lequel le dispositif de traitement de données (2) est réalisé de telle sorte que dans le cas d'un résultat de vérification positif, les positions résultant à l'issue du changement du dispositif de marquage sont soumises à une transformation qui annule le changement de la position au moins en partie, et que les signaux d'affichage sont calculés sur la base des positions transformées.

6. Système de navigation à marqueurs selon la revendication 5, dans lequel la transformation correspond à la transformation de vérification ou est calculée à partir de celle-ci.

7. Système de navigation à marqueurs selon l'une quelconque des revendications 1 à 3, dans lequel le dispositif de traitement de données est réalisé de telle sorte qu'il laisse les signaux d'affichage inchangés de telle sorte que le dispositif d'affichage n'affiche aucun changement des positions, ou que les changements déterminés sont affichés en partie seulement et/ou que les positions changées et déterminées sont soumises, avant la détermination des signaux d'affichage dans le cas d'un résultat de vérification positif, à la transformation de vérification de telle sorte que les changements sont annulés au moins en partie.

8. Système de navigation à marqueurs selon l'une quelconque des revendications 1 à 7, dans lequel le dispositif de traitement de données est réalisé pour déterminer la transformation de vérification sur la base de la supposition

que les changements déterminés aient été provoqués par une rotation et/ou une translation du dispositif de détection, et/ou

que les changements déterminés aient été provoqués par la rotation et/ou la translation d'un objet, par rapport auquel les dispositifs de marquage sont stationnaires.

9. Système de navigation à marqueurs selon l'une quelconque des revendications 1 à 8, dans lequel le dispositif de traitement de données (2) est réalisé pour :

déterminer la transformation de vérification en ce que respectivement une transformation de changement qui décrit le changement de la position entre le moment avant le changement et le moment après le changement pour chacun des dispositifs de marquage ;

comparer les transformations de changement calculées entre elles ; et

si toutes les transformations de changement sont similaires au moins dans une mesure prédéterminée, déterminer un résultat de vérification positif.

10. Système de navigation à marqueurs selon la revendication 9, dans lequel le dispositif de traitement de données (2) est réalisé pour déterminer la transformation de vérification à partir des transformations de changement.

11. Système de navigation à marqueurs selon l'une quelconque des revendications précédentes, dans lequel, dans le cas d'un résultat de vérification positif, la vitesse des changements est calculée, et

si la vitesse calculée est inférieure ou supérieure à une vitesse prédéterminée, les signaux d'affichage restent inchangés ou les signaux d'affichage sont déterminés de telle sorte que les changements déterminés ne sont pas affichés ou sont affichés en partie seulement.

12. Système de navigation à marqueurs selon la revendication 11, dans lequel, si la vitesse calculée est supérieure ou égale à la vitesse prédéterminée, les changements déterminés ne sont pas affichés ou sont affichés en partie seulement si lors de l'affichage des positions qui ont changé selon les changements déterminés, le dispositif d'affichage n'affiche plus toutes les positions représentées précédemment.

13. Procédé de détection et de représentation de la position de dispositifs de marquage au moyen d'un système de navigation à marqueurs, dans lequel les dispositifs de marquage sont fixés à des objets, en particulier à des structures corporelles et/ou des instruments médicaux, et dans lequel le procédé comprend les étapes suivantes consistant à :

détecter des signaux partant des dispositifs de marquage ;

calculer les positions des dispositifs de marquage (3a, 3b) sur la base des signaux détectés ;

déterminer des changements des positions des dispositifs de marquage sur la base des positions calculées ;

vérifier sur la base des changements déterminés si les changements de la position de chacun des dispositifs de marquage (3a, 3b) peuvent être décrits par une transformation de vérification qui est identique pour tous les changements, et qui transforme les positions des dispositifs de marquage (3a, 3b) avant le changement vers les positions des dispositifs de marquage (3a, 3b) après le changement au moins dans une mesure prédéterminée ;

déterminer des signaux d'affichage en fonction du résultat de vérification, les signaux représentant les positions des dispositifs de marquage (3a, 3b) et/ou des objets reliés aux dispositifs de marquage ;

afficher des signaux d'affichage correspondant aux positions.

EP 1 923 015 B1

Fig. 1

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19639615 A1
- US 6351659 B